# EUROPEAN PATENT APPLICATION

(11) **EP 4 817 629 A1**
(43) Date of publication of application: **07.10.2026**
(21) Application number: 24898233.2
(22) Date of filing: 29.11.2024
(51) Int. Cl.: A61K 39/395, A61K 9/08, A61K 47/18, A61K 47/12, A61K 47/22, A61K 47/20, A61K 47/26, A61K 9/00, A61P 17/00, A61P 11/00

(54) **STABLE SACCHARIDE-FREE LIQUID FORMULATION OF ANTI-IL-4R alpha ANTIBODY**

(30) Priority: 01.12.2023 KR 20230172609
(71) Applicant: Samsung Bioepis Co., Ltd., Yeonsu-gu Incheon 21987 (KR)
(72) Inventor: PARK, Jin Hye, Incheon 21982 (KR); KIM, Han Soo, Incheon 22001 (KR); LEE, Seung Ha, Incheon 21986 (KR); KIM, In Ae, Seoul 05090 (KR); LEE, Hun Joo, Incheon 21996 (KR); CHANG, Sung Keun, Incheon 22003 (KR)
(74) Representative: Boult Wade Tennant LLP
(86) International application number: PCT/KR2024/019361
(87) International publication number: WO 2025/116640

(57) **Abstract**

The present disclosure relates to a liquid formulation, which includes an anti-IL-4Rα antibody, a buffer, and an amino acid, is free of saccharide, and has a pH of 4.5 to 6.5, a device comprising the same, and a use thereof for treating an IL-4Rα-related condition. Despite being free of saccharide, the liquid formulation may secure the stability of the antibody while simultaneously improving the viscosity, and thus can be usefully used as a medicament for treating an IL-4Rα-related condition.

## Description

### Technical Field

The present disclosure relates to a stable saccharide-free liquid formulation of an anti-IL-4Rα antibody, a device comprising the same, and a use thereof for treating an IL-4Rα-related condition.

### Background Art

Antibody drugs have a large molecular weight and complex secondary/higher-order structures compared to general protein drugs, which may lead to physicochemical instability. For this reason, the development of an optimal formulation that guarantees the quality and stability of antibody drugs throughout the entire process of manufacture, storage, and administration to a patient is required. Protein instability can arise from various external factors such as temperature, light, and chemicals, which may reduce activity and decrease drug efficacy, or cause immunogenicity upon administration to the human body. Therefore, it is important to address such instability of antibody drugs to maintain optimal quality until they are administered to a patient. To achieve optimal quality, methods such as changing a buffer solution, evaluating an optimal pH, and adding a stabilizer are used. Because each antibody protein has different material properties, and the optimal combinations of a buffer solution, a pH, a stabilizer, and the like may vary, the development of a formulation suitable for the target material is required.

For example, dupilumab, an anti-IL-4Rα antibody, is manufactured and sold under the trade name Dupixent^{®}. Dupixent 200 mg is a 1.14 mL liquid preparation composed of 200 mg of an anti-IL-4Rα IgG, 1.2 mg of sodium acetate, 3.5 mg of histidine, 12 mg of arginine, 57 mg of sucrose, and 2.3 mg of polysorbate 80. Dupixent 300 mg is a 2.00 mL liquid preparation composed of 300 mg of an anti-IL-4Rα IgG, 2.0 mg of sodium acetate, 6.2 mg of histidine, 10.5 mg of arginine, 100 mg of sucrose, and 4 mg of polysorbate 80. For stabilization of a liquid preparation comprising various antibody proteins such as dupilumab, various conditions such as the type and concentration of each component are being reviewed and applied, such as adding a saccharide, an amino acid, a buffer, a surfactant, and the like, or applying a specific pH, as in the composition of the Dupixent product.

In addition to securing the stability described above, and in consideration of patient convenience during injection, there is a need to develop high-concentration preparations that offer increased therapeutic activity while reducing the number of administrations through small-volume, high-dose injections. In the formulation development of high-concentration preparations, increasing the protein concentration introduces limitations, such as aggregation-induced protein destabilization and impaired injectability resulting from elevated viscosity. Therefore, it is critical to develop a preparation that improves viscosity while ensuring protein stability. Although saccharides are commonly used as stabilizers for proteins, preparations containing such stabilizers tend to exhibit accelerated viscosity increases at higher protein concentrations, which limits the ability to simultaneously secure protein stability and improve viscosity. Therefore, the development of a preparation capable of addressing such limitations is required.

### Disclosure of Invention

### Technical Problem

The present disclosure relates to a stable saccharide-free liquid formulation of an anti-IL-4Rα antibody such as dupilumab. The saccharide-free liquid formulation exhibits improved stability and viscosity compared to a commercialized formulation of an anti-IL-4Rα antibody such as dupilumab (e.g., Dupixent) and is thus applicable to high-concentration preparations, and thus patient convenience may be increased.

One aspect is to provide a liquid formulation comprising an anti-IL-4Rα antibody, a buffer, and an amino acid, wherein the liquid formulation is free of saccharide and has a pH of about 4.5 to about 6.5.

Another aspect is to provide a device comprising the liquid formulation.

Another aspect is to provide a method of treating an IL-4Rα-related condition, comprising administering the liquid formulation to a subject in need thereof.

Another aspect is to provide a use of the liquid formulation in the manufacture of a medicament for treating an IL-4Rα-related condition.

### Solution to Problem

Unless otherwise defined, all technical terms used herein have the same meaning as commonly understood by a person of ordinary skill in the art to which the present disclosure pertains. In addition, although preferred methods or samples are described herein, those similar or equivalent thereto are also comprised within the scope of the present disclosure. Furthermore, the numerical values described herein are considered to comprise the meaning of "about" even when not explicitly stated. The contents of all publications cited as references herein are incorporated herein by reference in their entirety.

As used herein, the term "about" or "approximately" may generally be interpreted to comprise values or ranges within ± 10 %, within ± 5 %, within ± 4 %, within ± 3 %, within ± 2 %, or within ± 1 % of a given value or range.

One aspect provides a stable saccharide-free liquid formulation of an anti-IL-4Rα antibody, and specifically, provides a liquid formulation comprising:
an anti-IL-4Rα antibody;
a buffer; and
an amino acid,
wherein the liquid formulation is free of saccharide and
has a pH of about 4.5 to about 6.5.

### (1) Antibody

As used herein, the term "antibody" may be interpreted to comprise a full-length antibody or an antigen-binding fragment thereof. The antibody comprises a monoclonal antibody, a polyclonal antibody, a humanized antibody, a human antibody, and a chimeric antibody.

The term "antigen-binding fragment" refers to a fragment comprising an antigen-binding site of an antibody. For example, the antigen-binding fragment comprises, but is not limited to, a Fab fragment, an F(ab')₂ fragment, an Fc fragment, or an scFv fragment.

As used herein, the antibody may be an anti-IL-4Rα antibody. The anti-IL-4Rα antibody may refer to any antibody that binds to the interleukin-4 receptor alpha chain (IL-4Rα). The anti-IL-4Rα antibody can inhibit IL-4 and IL-13 signaling.

The anti-IL-4Rα antibody may be dupilumab (CAS No. 1190264-60-8). Therefore, the liquid formulation may be a stable liquid formulation of dupilumab. Dupilumab is a fully human monoclonal antibody that binds to IL-4Rα, and it belongs to IgG4. Dupilumab is sold under the trade name Dupixent^{®}. After being approved by the US Food and Drug Administration (FDA) as a treatment for atopic dermatitis, Dupixent is expanding its indications to various type 2 inflammatory diseases such as asthma, chronic rhinosinusitis with nasal polyposis (CRSwNP), and eosinophilic esophagitis (EoE). The sequence of dupilumab is known, and dupilumab may be produced by general methods known in the art. More detailed information on dupilumab may be easily obtained from known databases by a person skilled in the art.

As used herein, the term "dupilumab" may also be construed to encompass modified dupilumab in which the amino acid sequence is modified (by deletion, insertion, and/or substitution) and/or the glycosylation profile is modified, to an extent that it does not alter the polypeptide function.

The anti-IL-4Rα antibody may be comprised in the liquid formulation in a therapeutically effective amount.

The concentration of the anti-IL-4Rα antibody may be about 1 mg/mL to about 300 mg/mL, about 1 mg/mL to about 250 mg/mL, about 1 mg/mL to about 200 mg/mL, about 1 mg/mL to about 175 mg/mL, about 5 mg/mL to about 300 mg/mL, about 5 mg/mL to about 250 mg/mL, about 5 mg/mL to about 200 mg/mL, about 5 mg/mL to about 175 mg/mL, about 10 mg/mL to about 300 mg/mL, about 10 mg/mL to about 250 mg/mL, about 10 mg/mL to about 200 mg/mL, about 10 mg/mL to about 175 mg/mL, about 25 mg/mL to about 300 mg/mL, about 25 mg/mL to about 250 mg/mL, about 25 mg/mL to about 200 mg/mL, about 25 mg/mL to about 175 mg/mL, about 50 mg/mL to about 300 mg/mL, about 50 mg/mL to about 250 mg/mL, about 50 mg/mL to about 200 mg/mL, about 50 mg/mL to about 175 mg/mL, about 75 mg/mL to about 300 mg/mL, about 75 mg/mL to about 250 mg/mL, about 75 mg/mL to about 200 mg/mL, about 75 mg/mL to about 175 mg/mL, about 100 mg/mL to about 300 mg/mL, about 100 mg/mL to about 250 mg/mL, about 100 mg/mL to about 200 mg/mL, about 100 mg/mL to about 175 mg/mL, about 125 mg/mL to about 300 mg/mL, about 125 mg/mL to about 250 mg/mL, about 125 mg/mL to about 200 mg/mL, about 125 mg/mL to about 175 mg/mL, about 145 mg/mL to about 300 mg/mL, about 145 mg/mL to about 250 mg/mL, about 145 mg/mL to about 200 mg/mL, about 145 mg/mL to about 180 mg/mL, about 145 mg/mL to about 175 mg/mL, about 149 mg/mL to about 151 mg/mL, about 150 mg/mL to about 300 mg/mL, about 150 mg/mL to about 250 mg/mL, about 150 mg/mL to about 225 mg/mL, about 150 mg/mL to about 200 mg/mL, about 150 mg/mL to about 180 mg/mL, about 150 mg/mL to about 175 mg/mL, about 170 mg/mL to about 300 mg/mL, about 170 mg/mL to about 250 mg/mL, about 170 mg/mL to about 225 mg/mL, about 170 mg/mL to about 200 mg/mL, about 170 mg/mL to about 180 mg/mL, or about 174 mg/mL to about 176 mg/mL.

In an embodiment, the concentration of the anti-IL-4Rα antibody may be about 100 mg/mL to about 200 mg/mL.

In an embodiment, the concentration of the anti-IL-4Rα antibody may be about 125 mg/mL to about 200 mg/mL.

In an embodiment, the concentration of the anti-IL-4Rα antibody may be about 150 mg/mL to about 200 mg/mL.

In an embodiment, the concentration of the anti-IL-4Rα antibody may be about 150 mg/mL to about 175 mg/mL.

In an embodiment, the concentration of the anti-IL-4Rα antibody may be 135 mg/mL to 165 mg/mL.

In an embodiment, the concentration of the anti-IL-4Rα antibody may be 158 mg/mL to 192 mg/mL.

In a specific embodiment, the concentration of the anti-IL-4Rα antibody may be about 150 mg/mL.

In a specific embodiment, the concentration of the anti-IL-4Rα antibody may be about 175 mg/mL.

The concentration of the anti-IL-4Rα antibody may be a high concentration. Therefore, the liquid formulation may be a high-concentration liquid formulation of the anti-IL-4Rα antibody. The high concentration may mean 100 mg/mL or more or 150 mg/mL or more, for example, 100 mg/mL to 300 mg/mL or 150 mg/mL to 300 mg/mL, but is not limited thereto.

### (2) Buffer and pH

The liquid formulation according to one aspect comprises a buffer. The buffer may function to adjust the pH of the formulation. The pH of the liquid formulation may be maintained at a constant value or within a constant range by the buffer. Therefore, the buffer may serve to provide a constant value or a constant range of pH to the liquid formulation.

The buffer may be used without limitation in its type as long as it is applicable to biopharmaceuticals.

The buffer may comprise one or more selected from acetate, phosphate, citrate, succinate, malate, tartrate, carbonate, salts thereof, and hydrates thereof.

The term "salt" may refer to a pharmaceutically acceptable salt. The salt may comprise an inorganic acid salt, an organic acid salt, a metal salt, and the like of the compound. The inorganic acid salt may be a hydrochloride, hydrobromide, phosphate, sulfate, or disulfate. The organic acid salt may be a formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, a sodium salt, a magnesium salt, a strontium salt, or a potassium salt.

The term "hydrate" refers to a substance containing a water molecule within its molecule. The hydrate may be a monohydrate, a dihydrate, or a trihydrate.

The pH of the liquid formulation according to one aspect may be about 5.3 to about 6.5. The pH of the liquid formulation may be provided by the buffer. Alternatively, the pH of the liquid formulation may be adjusted by the addition of any acid (e.g., HCl) or base (e.g., NaOH).

Specifically, the pH of the formulation may be any range or any value selected within about 4.5 to about 6.5. For example, the pH may be about 4.5 to about 6.5, about 4.5 to about 6.0, about 4.5 to about 5.5, about 4.5 to about 5.3, about 4.5 to about 5.2, about 5.0 to about 6.5, about 5.0 to about 6.0, about 5.0 to about 5.5, about 5.0 to about 5.3, about 5.0 to about 5.2, about 5.1 to about 6.5, about 5.1 to about 6.0, about 5.1 to about 5.5, about 5.1 to about 5.3, about 5.1 to about 5.2, about 5.2 to about 6.5, about 5.2 to about 6.0, about 5.2 to about 5.5, about 5.2 to about 5.3, about 5.2 to about 5.2, about 5.3 to about 6.5, about 5.3 to about 6.4, about 5.3 to about 6.3, about 5.3 to about 6.2, about 5.3 to about 6.1, about 5.3 to about 6.0, about 5.4 to about 6.5, about 5.4 to about 6.4, about 5.4 to about 6.3, about 5.4 to about 6.2, about 5.4 to about 6.1, about 5.4 to about 6.0, about 5.5 to about 6.5, about 5.5 to about 6.4, about 5.5 to about 6.3, about 5.5 to about 6.2, about 5.5 to about 6.1, or about 5.5 to about 6.0.

In an embodiment, the pH of the liquid formulation may be about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5.

In an embodiment, the buffer may comprise one or more selected from acetate, salts thereof, and hydrates thereof. In an embodiment, the buffer may comprise acetate. In an embodiment, the buffer may be acetate. In an embodiment, the buffer may comprise sodium acetate. In an embodiment, the buffer may be sodium acetate.

The concentration of the buffer may be any range or any value selected within about 0.1 mM to about 50 mM. For example, the concentration of the buffer may be about 0.1 mM to about 50 mM, about 0.1 mM to about 40 mM, about 0.1 mM to about 30 mM, about 0.1 mM to about 20 mM, about 0.1 mM to about 15 mM, about 0.1 mM to about 14 mM, about 1 mM to about 50 mM, about 1 mM to about 40 mM, about 1 mM to about 30 mM, about 1 mM to about 20 mM, about 1 mM to about 15 mM, about 1 mM to about 14 mM, about 3 mM to about 50 mM, about 3 mM to about 40 mM, about 3 mM to about 30 mM, about 3 mM to about 20 mM, about 3 mM to about 15 mM, about 3 mM to about 14 mM, about 5 mM to about 50 mM, about 5 mM to about 40 mM, about 5 mM to about 30 mM, about 5 mM to about 20 mM, about 5 mM to about 15 mM, about 5 mM to about 14 mM, about 6 mM to about 50 mM, about 6 mM to about 40 mM, about 6 mM to about 30 mM, about 6 mM to about 20 mM, about 6 mM to about 15 mM, about 6 mM to about 14 mM, about 10 mM to about 50 mM, about 10 mM to about 40 mM, about 10 mM to about 35 mM, about 10 mM to about 30 mM, about 20 mM to about 50 mM, about 20 mM to about 40 mM, about 20 mM to about 35 mM, about 20 mM to about 30 mM, about 25 mM to about 50 mM, about 25 mM to about 40 mM, about 25 mM to about 35 mM, or about 25 mM to about 30 mM.

The concentration of the acetate may be any range or any value selected within about 0.1 mM to about 50 mM. For example, the concentration of the acetate may be about 0.1 mM to about 50 mM, about 0.1 mM to about 40 mM, about 0.1 mM to about 30 mM, about 0.1 mM to about 20 mM, about 0.1 mM to about 15 mM, about 0.1 mM to about 14 mM, about 1 mM to about 50 mM, about 1 mM to about 40 mM, about 1 mM to about 30 mM, about 1 mM to about 20 mM, about 1 mM to about 15 mM, about 1 mM to about 14 mM, about 3 mM to about 50 mM, about 3 mM to about 40 mM, about 3 mM to about 30 mM, about 3 mM to about 20 mM, about 3 mM to about 15 mM, about 3 mM to about 14 mM, about 5 mM to about 50 mM, about 5 mM to about 40 mM, about 5 mM to about 30 mM, about 5 mM to about 20 mM, about 5 mM to about 15 mM, about 5 mM to about 14 mM, about 6 mM to about 50 mM, about 6 mM to about 40 mM, about 6 mM to about 30 mM, about 6 mM to about 20 mM, about 6 mM to about 15 mM, about 6 mM to about 14 mM, about 10 mM to about 50 mM, about 10 mM to about 40 mM, about 10 mM to about 35 mM, about 10 mM to about 30 mM, about 20 mM to about 50 mM, about 20 mM to about 40 mM, about 20 mM to about 35 mM, about 20 mM to about 30 mM, about 25 mM to about 50 mM, about 25 mM to about 40 mM, about 25 mM to about 35 mM, or about 25 mM to about 30 mM.

In an embodiment, the concentration of the acetate may be about 6 mM to about 14 mM, about 6 mM to about 12 mM, about 6 mM to about 10 mM, about 6 mM to about 8 mM, about 8 mM to about 14 mM, about 8 mM to about 12 mM, about 8 mM to about 10 mM, about 10 mM to about 14 mM, about 10 mM to about 12 mM, or about 12 mM to about 14 mM.

In an embodiment, the concentration of the acetate may be about 10 mM to about 50 mM, about 10 mM to about 40 mM, about 10 mM to about 35 mM, about 10 mM to about 30 mM, about 20 mM to about 50 mM, about 20 mM to about 40 mM, about 20 mM to about 35 mM, about 20 mM to about 30 mM, about 25 mM to about 50 mM, about 25 mM to about 40 mM, about 25 mM to about 35 mM, or about 25 mM to about 30 mM.

### (3) Saccharide

The liquid formulation according to one aspect is free of saccharide. Therefore, the liquid formulation may be a saccharide-free formulation.

The liquid formulation may have improved stability and improved viscosity compared to a commercialized formulation, despite being a saccharide-free formulation.

The term "free of component A" or "substantially free of component A" may be interpreted to comprise a case where component A is not present at all, a case where, even when component A is present, it is present in a trace amount that does not substantially affect the properties of the formulation, or a case where it is present in an undetectable amount.

As used herein, the phrase "free of saccharide" may be interpreted to mean that saccharide component is not present in the formulation, or that a saccharide is comprised in an amount incapable of exhibiting an intended function as a stabilizer in the formulation.

The saccharide may comprise a sugar and a sugar alcohol. Therefore, the liquid formulation may not comprise either a sugar or a sugar alcohol.

The sugar may be a monosaccharide, a disaccharide, an oligosaccharide, or a polysaccharide. The sugar may comprise one or more selected from sucrose, trehalose, galactose, mannose, maltose, lactose, fructose, and glucose.

The sugar alcohol, which is obtained by reducing an aldehyde group or a ketone group of a sugar to an alcohol group, is a generic term for a polyol having two or more hydroxy groups. The sugar alcohol may comprise one or more selected from mannitol, sorbitol, xylitol, arabitol, erythritol, lactitol, maltitol, and inositol. The sugar alcohol comprises an anhydrous form or a hydrate of the sugar alcohol. For example, trehalose may comprise not only trehalose but also trehalose dihydrate.

In an embodiment, the liquid formulation may not comprise any of sucrose, trehalose, sorbitol, and mannitol.

### (4) Amino Acid

The liquid formulation according to one aspect comprises an amino acid.

The amino acid may comprise one or more selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

In an embodiment, the amino acid may comprise one or more selected from arginine, histidine, glycine, methionine, and lysine.

In an embodiment, the amino acid may be selected from the following:
1) arginine;
2) a combination of arginine and lysine;
3) a combination of arginine and methionine;
4) histidine;
5) a combination of histidine and lysine;
6) a combination of histidine and methionine;
7) glycine;
8) a combination of glycine and lysine;
9) a combination of glycine and methionine; or
10) a combination of histidine, arginine, and methionine.

The concentration of the amino acid may be any range or any value selected within about 20 mM to about 355 mM.

In an embodiment, the concentration of the arginine may be about 75 mM to about 165 mM, about 75 mM to about 150 mM, about 75 mM to about 135 mM, about 75 mM to about 125 mM, about 75 mM to about 115 mM, about 75 mM to about 105 mM, about 75 mM to about 100 mM, about 90 mM to about 165 mM, about 90 mM to about 150 mM, about 90 mM to about 135 mM, about 90 mM to about 125 mM, about 90 mM to about 115 mM, about 90 mM to about 105 mM, about 90 mM to about 100 mM, about 95 mM to about 165 mM, about 95 mM to about 150 mM, about 95 mM to about 135 mM, about 95 mM to about 125 mM, about 95 mM to about 115 mM, about 95 mM to about 105 mM, about 95 mM to about 100 mM, about 100 mM to about 165 mM, about 100 mM to about 150 mM, about 100 mM to about 135 mM, about 100 mM to about 125 mM, about 100 mM to about 115 mM, about 100 mM to about 105 mM, about 105 mM to about 165 mM, about 105 mM to about 150 mM, about 105 mM to about 135 mM, about 105 mM to about 125 mM, about 115 mM to about 165 mM, about 115 mM to about 150 mM, about 115 mM to about 135 mM, or 115 mM to about 125 mM. In a specific embodiment, the concentration of the arginine may be about 100 mM or about 120 mM.

In an embodiment, the concentration of the histidine may be about 85 mM to about 205 mM, about 85 mM to about 175 mM, about 85 mM to about 145 mM, about 85 mM to about 115 mM, about 115 mM to about 205 mM, about 115 mM to about 175 mM, about 115 mM to about 145 mM, about 145 mM to about 205 mM, about 145 mM to about 175 mM, or about 140 mM to about 150 mM. In a specific embodiment, the concentration of the histidine may be about 145 mM.

In an embodiment, the concentration of the histidine may be about 30 mM to about 70 mM, about 30 mM to about 60 mM, about 30 mM to about 55 mM, about 30 mM to about 50 mM, 40 mM to about 70 mM, about 40 mM to about 60 mM, about 40 mM to about 55 mM, about 40 mM to about 50 mM, 45 mM to about 70 mM, about 45 mM to about 60 mM, about 45 mM to about 55 mM, about 45 mM to about 50 mM, 50 mM to about 70 mM, about 50 mM to about 60 mM, or about 50 mM to about 55 mM. In a specific embodiment, the concentration of the histidine may be about 50 mM.

The concentration of the glycine may be about 125 mM to about 315 mM, about 125 mM to about 290 mM, about 125 mM to about 270 mM, about 125 mM to about 250 mM, about 125 mM to about 230 mM, about 150 mM to about 315 mM, about 150 mM to about 290 mM, about 150 mM to about 270 mM, about 150 mM to about 250 mM, about 150 mM to about 230 mM, about 170 mM to about 315 mM, about 170 mM to about 290 mM, about 170 mM to about 270 mM, about 170 mM to about 250 mM, about 170 mM to about 230 mM, about 190 mM to about 315 mM, about 190 mM to about 290 mM, about 190 mM to about 270 mM, about 190 mM to about 250 mM, about 190 mM to about 230 mM, about 210 mM to about 315 mM, about 210 mM to about 290 mM, about 210 mM to about 270 mM, about 210 mM to about 250 mM, or about 210 mM to about 230 mM. In a specific embodiment, the concentration of the glycine may be about 220 mM.

The concentration of the methionine may be about 10 mM to about 40 mM, about 10 mM to about 35 mM, about 10 mM to about 30 mM, about 10 mM to about 25 mM, about 20 mM to about 40 mM, about 20 mM to about 35 mM, about 20 mM to about 30 mM, about 20 mM to about 25 mM, about 25 mM to about 40 mM, about 25 mM to about 35 mM, or about 25 mM to about 30 mM. In a specific embodiment, the concentration of the methionine may be about 25 mM or about 30 mM.

The concentration of the lysine may be about 20 mM to about 40 mM, about 20 mM to about 35 mM, about 20 mM to about 30 mM, about 25 mM to about 40 mM, about 25 mM to about 35 mM, or about 25 mM to about 30 mM. In a specific embodiment, the concentration of the lysine may be about 30 mM.

In an embodiment, the amino acid may be a combination of histidine and methionine. In this case, the concentration of the histidine may be about 85 mM to about 205 mM, and the concentration of the methionine may be about 20 mM to about 40 mM.

In an embodiment, the amino acid may be a combination of histidine, arginine, and methionine. In this case, the concentration of the histidine may be about 30 mM to about 70 mM, the concentration of the arginine may be about 80 mM to about 120 mM, and the concentration of the methionine may be about 10 mM to about 40 mM.

The amino acid may have one or more functions of a viscosity reducing agent and a stabilizer. The amino acid may have all the functions of a viscosity reducing agent and a stabilizer.

The term "viscosity reducing agent" or "viscosity reducer" refers to a substance that lowers the viscosity of a liquid substance.

The term "stabilizer" refers to a substance added to prevent a state change or a chemical change when preserving a substance. The stabilizer may be a thermal stabilizer. The stabilizer may inhibit aggregation of the antibody.

By including a specific type of amino acid or a specific combination of amino acids instead of saccharide, the liquid formulation may have an improved viscosity compared to a saccharide-containing anti-IL-4Rα antibody liquid formulation.

By including a specific type of amino acid or a specific combination of amino acids instead of saccharide, the liquid formulation may decrease the high molecular weight species content (%HMW) of the antibody in the liquid formulation.

By including a specific type of amino acid or a specific combination of amino acids instead of saccharide, the %HMW of the antibody in the liquid formulation may be decreased immediately after preparation or upon storage under the same conditions (e.g., at 25 °C for 4 weeks) compared to a saccharide-containing anti-IL-4Rα antibody liquid formulation.

By including a specific type of amino acid or a specific combination of amino acids instead of saccharide, both viscosity and %HMW of the liquid formulation are decreased compared to saccharide-containing Dupixent, and thus viscosity and stability may be simultaneously improved.

### (5) Surfactant

The liquid formulation according to one aspect may further comprise a surfactant.

Therefore, the liquid formulation may be a liquid formulation comprising an anti-IL-4Rα antibody, a buffer, an amino acid, and a surfactant, wherein the liquid formulation is free of saccharide and has a pH of about 5.3 to about 6.5.

The surfactant may be selected from any pharmaceutically acceptable surfactants capable of evenly dispersing a protein (e.g., an antibody) in a liquid formulation medium.

The surfactant may be a nonionic surfactant.

Specifically, the surfactant may be one or more selected from the group consisting of a polysorbate, a poloxamer, a sorbitan ester of another fatty acid, a polyethylenepolypropylene glycol, a polyoxyethylene compound, sodium dodecyl sulphate (SDS), and the like.

The polysorbate may comprise polysorbate 20, polysorbate 40, polysorbate 60, polysorbate 65, polysorbate 80, and polysorbate 85, and the like.

The poloxamer may comprise a PEO-PPO-PEO copolymer (PEO is poly(ethylene oxide) and PPO is poly(propylene oxide)), and the like.

The sorbitan ester of another fatty acid may refer to a sorbitan ester of a fatty acid other than a polysorbate, and may comprise, for example, a sorbitan polyethoxylate, and the like.

The polyoxyethylene compound may comprise polyoxyethylene stearate, a polyoxyethylene alkyl ether (alkyl: C1-C30), polyoxyethylene monolauryl ether, an alkylphenyl polyoxyethylene copolymer (alkyl: C1-C30), and the like.

In an embodiment, the surfactant may be a polysorbate.

In an embodiment, the surfactant may comprise one or more selected from polysorbate 20, polysorbate 40, polysorbate 60, polysorbate 65, polysorbate 80, and polysorbate 85.

In an embodiment, the surfactant may comprise polysorbate 20, polysorbate 80, or a combination thereof.

In a specific embodiment, the surfactant may comprise polysorbate 80. In a specific embodiment, the surfactant may be polysorbate 80.

The concentration of the surfactant may be any range or any value selected within about 0.01% (w/v) to about 0.9% (w/v). For example, the concentration of the surfactant may be about 0.01% (w/v) to about 0.9% (w/v), about 0.01% (w/v) to about 0.5% (w/v), about 0.1% (w/v) to about 0.9% (w/v), about 0.1% (w/v) to about 0.5% (w/v), about 0.1% (w/v) to about 0.4% (w/v), about 0.1% (w/v) to about 0.3% (w/v), about 0.15% (w/v) to about 0.9% (w/v), about 0.15% (w/v) to about 0.5% (w/v), about 0.15% (w/v) to about 0.4% (w/v), about 0.15% (w/v) to about 0.3% (w/v), or about 0.15% (w/v) to about 0.25% (w/v).

In a specific embodiment, the concentration of the surfactant may be about 0.2% (w/v).

### (6) Diluent

The liquid formulation according to one aspect may further comprise a diluent.

The diluent may be an aqueous carrier. The aqueous carrier is a pharmaceutically acceptable carrier that is safe and non-toxic when it is administered to a human, and may be, for example, water, a saline solution, a Ringer's solution, dextrose, or a mixture thereof.

In an embodiment, the diluent may be water. Therefore, the liquid formulation may be an aqueous liquid formulation.

### (7) Formulation

The term "liquid formulation" refers to a formulation in a liquid state.

The liquid formulation according to one aspect is a stable liquid formulation of an anti-IL-4Rα antibody.

The liquid formulation according to one aspect may be a pharmaceutical formulation of an anti-IL-4Rα antibody.

The term "pharmaceutical composition" or "pharmaceutical preparation" refers to a preparation that allows the biological activity of an active ingredient to act effectively, and does not include an additional ingredient having severe toxicity to a subject to which the preparation is to be administered.

The term "pharmaceutical formulation" refers to a product of a process of producing a final drug product by combining an active drug with a chemical substance.

The term "pharmaceutically acceptable" may refer to excipients, carriers, vehicles, diluents, additives, salts, and the like suitable for administration to a subject.

The liquid formulation according to one aspect may be a Dupixent^{®} biosimilar. The liquid formulation according to one aspect has improved stability and improved viscosity compared to Dupixent.

The term "biosimilar", also called a "biogeneric", refers to a generic drug for an original biopharmaceutical. A biopharmaceutical is produced through cells rather than synthesized as a chemical product, and thus a product perfectly identical to the original drug cannot be replicated. Therefore, a generic drug for a biopharmaceutical is called a biosimilar in the sense that it is similar to, although not identical to, the original drug.

The liquid formulation according to one aspect may be selected from the following items:
1) a liquid formulation comprising an anti-IL-4Rα antibody, a buffer, and an amino acid, free of saccharide, and having a pH of about 4.5 to about 6.5;
2) a liquid formulation comprising an anti-IL-4Rα antibody, a buffer, an amino acid, and a surfactant, free of saccharide, and having a pH of about 4.5 to about 6.5;
3) a liquid formulation comprising an anti-IL-4Rα antibody, a buffer comprising acetate, an amino acid comprising arginine, and a surfactant, free of saccharide, and having a pH of about 4.5 to about 6.5;
4) a liquid formulation comprising an anti-IL-4Rα antibody, a buffer comprising acetate, an amino acid comprising arginine and lysine, and a surfactant, free of saccharide, and having a pH of about 4.5 to about 6.5;
5) a liquid formulation comprising an anti-IL-4Rα antibody, a buffer comprising acetate, an amino acid comprising arginine and methionine, and a surfactant, free of saccharide, and having a pH of about 4.5 to about 6.5;
6) a liquid formulation comprising an anti-IL-4Rα antibody, a buffer comprising acetate, an amino acid comprising histidine, and a surfactant, free of saccharide, and having a pH of about 4.5 to about 6.5;
7) a liquid formulation comprising an anti-IL-4Rα antibody, a buffer comprising acetate, an amino acid comprising histidine and lysine, and a surfactant, free of saccharide, and having a pH of about 4.5 to about 6.5;
8) a liquid formulation comprising an anti-IL-4Rα antibody, a buffer comprising acetate, an amino acid comprising histidine and methionine, and a surfactant, free of saccharide, and having a pH of about 4.5 to about 6.5;
9) a liquid formulation comprising an anti-IL-4Rα antibody, a buffer comprising acetate, an amino acid comprising glycine, and a surfactant, free of saccharide, and having a pH of about 4.5 to about 6.5;
10) a liquid formulation comprising an anti-IL-4Rα antibody, a buffer comprising acetate, an amino acid comprising glycine and lysine, and a surfactant, free of saccharide, and having a pH of about 4.5 to about 6.5;
11) a liquid formulation comprising an anti-IL-4Rα antibody, a buffer comprising acetate, an amino acid comprising glycine and methionine, and a surfactant, free of saccharide, and having a pH of about 4.5 to about 6.5;
12) a liquid formulation comprising about 100 mg/mL to about 200 mg/mL of dupilumab, about 6 mM to about 14 mM of acetate, about 20 mM to about 355 mM of an amino acid, the amino acid comprising one or more selected from arginine, histidine, glycine, methionine, and lysine, and about 0.1% (w/v) to about 0.9% (w/v) of polysorbate 80, free of saccharide, and having a pH of about 4.5 to about 6.5;
13) a liquid formulation comprising about 100 mg/mL to about 200 mg/mL of dupilumab, about 6 mM to about 14 mM of acetate, about 85 mM to about 205 mM of histidine, about 20 mM to about 40 mM of methionine, and about 0.1% (w/v) to about 0.9% (w/v) of polysorbate 80, free of saccharide, and having a pH of about 4.5 to about 6.5;
14) a liquid formulation comprising about 100 mg/mL to about 200 mg/mL of dupilumab, 6 mM±1 mM of acetate, 145 mM±20 mM of histidine, 30 mM±2 mM of methionine, and 0.2%±0.1% (w/v) of polysorbate 80, free of saccharide, and having a pH of 5.9±0.2;
15) a liquid formulation comprising about 100 mg/mL to about 200 mg/mL of dupilumab, 8 mM±1 mM of acetate, 115 mM to 175 mM of histidine, 30 mM±2 mM of methionine, and 0.2%±0.1% (w/v) of polysorbate 80, free of saccharide, and having a pH of 4.5 to 6.2;
16) a liquid formulation comprising about 100 mg/mL to about 200 mg/mL of dupilumab, 10 mM±1 mM of acetate, 85 mM to 205 mM of histidine, 20 mM to 40 mM of methionine, and 0.2%±0.1% (w/v) of polysorbate 80, free of saccharide, and having a pH of 4.5 to 6.5;
17) a liquid formulation comprising about 100 mg/mL to about 200 mg/mL of dupilumab, 12 mM±1 mM of acetate, 115 mM to 175 mM of histidine, 25 mM to 35 mM of methionine, and 0.2%±0.1% (w/v) of polysorbate 80, free of saccharide, and having a pH of 4.5 to 6.2;
18) a liquid formulation comprising about 100 mg/mL to about 200 mg/mL of dupilumab, 14 mM±1 mM of acetate, 145 mM±20 mM of histidine, 30 mM±2 mM of methionine, and 0.2%±0.1% (w/v) of polysorbate 80, free of saccharide, and having a pH of 5.9±0.2;
19) a liquid formulation comprising about 100 mg/mL to about 200 mg/mL of dupilumab, about 20 mM to about 40 mM of acetate, about 40 mM to about 60 mM of histidine, about 80 mM to about 120 mM of arginine, about 10 mM to about 40 mM of methionine, and about 0.1% (w/v) to about 0.9% (w/v) of polysorbate 80, free of saccharide, and having a pH of about 4.5 to about 5.9; or
20) a liquid formulation comprising about 100 mg/mL to about 200 mg/mL of dupilumab, 30 mM±5 mM of acetate, 50 mM±5 mM of histidine, 100 mM±5 mM of arginine, 25 mM±5 mM of methionine, and about 0.1% (w/v) to about 0.9% (w/v) of polysorbate 80, free of saccharide, and having a pH of 5.2±0.2.
(8) Stability and Viscosity

The liquid formulation according to one aspect may secure the stability of the antibody while simultaneously having a low viscosity.

In the Examples of the present disclosure, two experiments were performed. First, it was confirmed that the saccharide-free formulation has improved viscosity and improved stability compared to the saccharide-containing formulation by comparatively evaluating viscosity and high molecular weight species content (%HMW). Second, through a comparative evaluation of viscosity and %HMW with the Dupixent formulation, it was confirmed that various saccharide-free anti-IL-4Rα antibody liquid formulations having different buffer concentrations, amino acid concentrations, and pH values simultaneously improved stability and viscosity compared to a commercialized formulation.

The liquid formulation may have an improved viscosity. The 'improvement' of the viscosity may mean a 'decrease' in the viscosity. Therefore, the liquid formulation may have a decreased viscosity. The unit of the viscosity may be expressed in cP (centipoise).

The saccharide-free liquid formulation may have an improved viscosity compared to a saccharide-containing anti-IL-4Rα antibody liquid formulation

The viscosity of the saccharide-free liquid formulation may be decreased by 5 % or more, 10 % or more, 20 % or more, 30 % or more, 40 % or more, 50 % or more, or 60 % or more compared to the viscosity of a saccharide-containing anti-IL-4Rα antibody liquid formulation.

The viscosity of the saccharide-free liquid formulation may be less than 17.0 cP, 16.8 cP or less, 16.0 cP or less, 15.0 cP or less, 14.0 cP or less, 13.0 cP or less, 12.0 cP or less, 11.0 cP or less, 10.0 cP or less, 9.0 cP or less, 8.0 cP or less, or 7.0 cP or less.

The liquid formulation according to one aspect may have a decreased viscosity compared to Dupixent. In an embodiment, the liquid formulation according to one aspect may have a decreased viscosity compared to a viscosity of 11.1 cP (centipoise) of Dupixent 200 mg. In another specific embodiment, the liquid formulation according to one aspect may have a decreased viscosity compared to a viscosity of Dupixent 300 mg.

In an embodiment, a saccharide-free liquid formulation including an amino acid selected from the following may have a viscosity decreased by 5 % or more, 10 % or more, 15 % or more, 20 % or more, 25 % or more, or 30 % or more compared to the viscosity of the Dupixent formulation:
1) arginine;
2) a combination of arginine and lysine;
3) a combination of arginine and methionine;
4) histidine;
5) a combination of histidine and lysine;
6) a combination of histidine and methionine;
7) glycine;
8) a combination of glycine and lysine;
9) a combination of glycine and methionine; or
10) a combination of histidine, arginine, and methionine.

In an embodiment, a saccharide-free liquid formulation including an amino acid combination of histidine and methionine may have a viscosity decreased by 5 % or more, 10 % or more, 15 % or more, 20 % or more, 25 % or more, or 30 % or more compared to the viscosity of the Dupixent formulation.

Therefore, the viscosity of the liquid formulation may be less than 11.1 cP. The viscosity of the liquid formulation may be less than 11.1 cP, 11.0 cP or less, 10.5 cP or less, 10.0 cP or less, 9.5 cP or less, 9.0 cP or less, 8.5 cP or less, 8.0 cP or less, 7.5 cP or less, 7.0 cP or less, or 6.5 cP or less. The viscosity of the liquid formulation may be 5.0 cP or more to less than 11.1 cP, 5.0 cP to 11.0 cP, 5.0 cP to 10.5 cP, 5.0 cP to 10.0 cP, 5.0 cP to 9.5 cP, 5.0 cP to 9.0 cP, 5.0 cP to 8.5 cP, 5.0 cP to 8.0 cP, 5.0 cP to 7.5 cP, 5.0 cP to 7.0 cP, 5.0 cP to 6.5 cP, 5.5 cP or more to 11.1 cP or less, 5.5 cP to 11.0 cP, 5.5 cP to 10.5 cP, 5.5 cP to 10.0 cP, 5.5 cP to 9.5 cP, 5.5 cP to 9.0 cP, 5.5 cP to 8.5 cP, 5.5 cP to 8.0 cP, 5.5 cP to 7.5 cP, 5.5 cP to 7.0 cP, 5.5 cP to 6.5 cP, 6.0 cP or more to 11.1 cP or less, 6.0 cP to 11.0 cP, 6.0 cP to 10.5 cP, 6.0 cP to 10.0 cP, 6.0 cP to 9.5 cP, 6.0 cP to 9.0 cP, 6.0 cP to 8.5 cP, 6.0 cP to 8.0 cP, 6.0 cP to 7.5 cP, 6.0 cP to 7.0 cP, 6.0 cP to 6.5 cP, 6.5 cP or more to 11.1 cP or less, 6.5 cP to 11.0 cP, 6.5 cP to 10.5 cP, 6.5 cP to 10.0 cP, 6.5 cP to 9.5 cP, 6.5 cP to 9.0 cP, 6.5 cP to 8.5 cP, 6.5 cP to 8.0 cP, 6.5 cP to 7.5 cP, 6.5 cP to 7.0 cP, 7.0 cP or more to 11.1 cP or less, 7.0 cP to 11.0 cP, 7.0 cP to 10.5 cP, 7.0 cP to 10.0 cP, 7.0 cP to 9.5 cP, 7.0 cP to 9.0 cP, 7.0 cP to 8.5 cP, 7.0 cP to 8.0 cP, 7.5 cP or more to 11.1 cP or less, 7.5 cP to 11.0 cP, 7.5 cP to 10.5 cP, 7.5 cP to 10.0 cP, 7.5 cP to 9.5 cP, 7.5 cP to 9.0 cP, 7.5 cP to 8.5 cP, 7.5 cP to 8.0 cP, 8.0 cP or more to 11.1 cP or less, 8.0 cP to 11.0 cP, 8.0 cP to 10.5 cP, 8.0 cP to 10.0 cP, 8.0 cP to 9.5 cP, 8.0 cP to 9.0 cP, or 8.0 cP to 8.5 cP.

The term "stability" means that the antibody (e.g., dupilumab) included in the formulation substantially retains its physical stability, chemical stability, and/or biological activity before and after administration, during an additional manufacturing process, or upon preservation or storage. For example, it may be understood to mean that the degree of loss of stability, such as aggregation, degradation, denaturation (acidic or basic), oxidation, and the like of the antibody included in the formulation, is 20 % or less, 15 % or less, 10 % or less, or 5 % or less compared to the initial stage of storage. Therefore, "excellent stability" or "improved stability" may mean a low protein aggregation rate, a low protein degradation rate, a low protein denaturation rate, a low amino acid oxidation rate, and the like during storage. Physical stability, chemical stability, and/or biological activity may be evaluated by commonly known methods.

The term "aggregate" may refer to a high molecular weight (HMW) species formed by aggregation of an antibody protein. The term "protein aggregation rate" may be expressed as a high molecular weight species content (%HMW) of the antibody in the formulation at a certain time point. The %HMW may be measured by size exclusion chromatography (SEC), but is not limited thereto. For example, "improved stability" may mean that the %HMW of the antibody measured for the formulation is decreased by 0.01 % or more, 0.1 % or more, 0.5 % or more, 1 % or more, 2 % or more, 3 % or more, 4 % or more, 5 % or more, 10 % or more, 15 % or more, or 20 % or more compared to an existing formulation. A decrease in %HMW may mean that the stability is improved as the degree of aggregation of the antibody in the formulation is decreased.

The stability evaluation may be performed immediately after preparation of the formulation, or after storage for a certain period of time under accelerated stability conditions or stress stability conditions. The accelerated stability conditions may include conditions used in an accelerated test for pharmaceuticals, and may be, for example, a temperature of 25±2 °C and a relative humidity (RH) of 60±5 %. The stress stability conditions may include conditions used in a stress test for pharmaceuticals, and may be, for example, a temperature of 40±2 °C and a relative humidity (RH) of 75 + 5 %.

The liquid formulation may have a decreased high molecular weight species content (%HMW).

The saccharide-free liquid formulation may have a decreased %HMW compared to a saccharide-containing anti-IL-4Rα antibody liquid formulation.

In the saccharide-free liquid formulation, the %HMW of the antibody measured after storage at 25 °C for 4 weeks may be decreased by 1 % or more, 2 % or more, 3 % or more, 4 % or more, 5 % or more, 10 % or more, 12 % or more, 15 % or more, or 20 % or more compared to a saccharide-containing formulation.

In the saccharide-free liquid formulation, the %HMW of the antibody measured after storage at 25 °C for 4 weeks may be less than 3.70 %, 3.62 % or less, 3.50 % or less, 3.40 % or less, 3.30 % or less, 3.20 % or less, 3.10 % or less, 3.00 % or less, 2.90 % or less, 2.80 % or less, 2.70 % or less, 2.60 % or less, 2.50 % or less, 2.40 % or less, 2.30 % or less, 2.20 % or less, or 2.10 % or less.

The saccharide-free liquid formulation according to one aspect may have a decreased %HMW compared to Dupixent. In an embodiment, the saccharide-free liquid formulation according to one aspect may have a decreased %HMW compared to Dupixent 200 mg, in which the %HMW of the antibody measured immediately after preparation is 2.03 %.

In an embodiment, in a saccharide-free liquid formulation including an amino acid combination of histidine and methionine, the %HMW of the antibody measured immediately after preparation may be decreased by 2 % or more, 5 % or more, 10 % or more, 15 % or more, or 20 % or more compared to the Dupixent formulation.

In the saccharide-free liquid formulation, the %HMW of the antibody measured immediately after preparation may be less than 2.03 %, 2.00 % or less, 1.90 % or less, 1.80 % or less, or 1.70 % or less.

### (9) Device

Another aspect provides a device comprising the liquid formulation according to one aspect.

The device is mainly used for a purpose of parenteral administration. The parenteral administration may include, for example, subcutaneous, intramuscular, intravenous, intraperitoneal, intracerebrospinal, intra-articular, intrasynovial, or intrathecal administration. The device may be accompanied by instructions for administration.

The device may comprise the liquid formulation in a container selected from a syringe, a pre-filled syringe, a pre-filled pen, a microinfuser, an autoinjector, a bottle, a vial, and a tube, but is not limited thereto.

The device may be in a single-dose form or a multiple-dose form.

In an embodiment, the device may comprise the liquid formulation in a pre-filled syringe. The pre-filled syringe may be a single-dose pre-filled syringe.

In an embodiment, the device may comprise the liquid formulation in a pre-filled pen. The pre-filled pen may be a single-dose pre-filled pen.

### (10) Treatment of Disease

Another aspect provides a method of treating an IL-4Rα-related condition, the method comprising administering the liquid formulation according to one aspect to a subject in need thereof. The liquid formulation may be in a form included in a device.

Another aspect provides a use of the liquid formulation according to one aspect in the manufacture of a medicament for treating an IL-4Rα-related condition.

The method for treating an IL-4Rα-related condition may further comprise identifying a subject in need of administration of an anti-IL-4Rα antibody (e.g., dupilumab) prior to the administering step.

The subject may be a subject in need of administration of a liquid formulation comprising the anti-IL-4Rα antibody (e.g., dupilumab). The subject in need of administration of the liquid formulation comprising the anti-IL-4Rα antibody (e.g., dupilumab) may be a subject having a disease or disorder that may be significantly treated (e.g., elimination, mitigation, alleviation, or improvement of symptoms, and the like) by administration of the anti-IL-4Rα antibody. The subject may be selected from mammals comprising humans.

The liquid formulation may be administered in a pharmaceutically effective amount.

The IL-4Rα-related condition may include any condition, disease, or disorder that may be treated by administration of the anti-IL-4Rα antibody. The IL-4Rα-related condition that may be treated by administration of an anti-IL-4Rα antibody (e.g., dupilumab) may include all indications previously approved or all indications that may be approved in the future for the anti-IL-4Rα antibody (e.g., dupilumab).

The IL-4Rα-related condition may be an IL-4-mediated disease and/or an IL-13-mediated disease. The IL-4-mediated disease may include any disease that may be treated by inhibition of IL-4 signaling. The IL-13-mediated disease may include any disease that may be treated by inhibition of IL-13 signaling. The IL-4Rα-related condition may include any disease that may be treated by a dual blockade of IL-4 and IL-13.

The IL-4Rα-related condition may include an inflammatory disease, an allergic disease, or an autoimmune disease. The inflammatory disease may be a Type 2 inflammatory disease.

The IL-4Rα-related condition may be any one selected from atopic dermatitis, asthma, chronic rhinosinusitis (CRS), chronic rhinosinusitis with nasal polyposis (CRSwNP), allergic fungal rhinosinusitis (AFRS), eosinophilic esophagitis (EoE), prurigo nodularis (PN), bullous pemphigoid (BP), chronic spontaneous urticaria (CSU), chronic inducible urticaria (CIndU), and chronic obstructive pulmonary disease (COPD), but is not limited thereto.

The atopic dermatitis may be moderate-to-severe atopic dermatitis.

The asthma may be moderate-to-severe asthma.

### (11) Route of Administration and Dosage

The liquid formulation according to one aspect may be administered by a parenteral route. The parenteral route may include subcutaneous administration, intravenous administration, and the like. The parenteral administration may be by a bolus injection or a continuous infusion.

In a specific embodiment, the liquid formulation may be for subcutaneous injection.

The liquid formulation may be formulated into a formulation suitable for the route of administration. For example, the liquid formulation may be formulated as an injection, an injectable ready-to-use form, and the like, but is not limited thereto.

The liquid formulation may be formulated such that the total amount or a pharmaceutically effective amount of an anti-IL-4Rα antibody (e.g., dupilumab) is provided in a single unit dosage form, or is divided across two or more (e.g., 2, 3, 4, 5, 6, 7, 8, 9, or 10) unit dosage forms. The liquid formulation may be included in a single-dose or multiple-dose form of a device.

The liquid formulation may be administered such that the total amount of the anti-IL-4Rα antibody (e.g., dupilumab) included in one unit dosage form is administered in vivo as a single bolus (e.g., within 1 minute, within 30 seconds, within 20 seconds, or within 10 seconds); or is administered as a gradual infusion in vivo over 5 minutes or more, 10 minutes or more, 30 minutes or more, 60 minutes or more, 90 minutes or more, 120 minutes or more, 150 minutes or more, 180 minutes or more, 210 minutes or more, or 240 minutes or more, but is not limited thereto.

A subject to be administered the liquid formulation may be selected from mammals including primates (e.g., humans, etc.), rodents (e.g., mice, rats, guinea pigs, hamsters, rabbits, etc.), cats, dogs, pigs, cows, horses, and the like.

The pharmaceutically effective amount of the liquid formulation or the anti-IL-4Rα antibody (e.g., dupilumab) included therein may mean an amount or dose sufficient to elicit a desired pharmacological effect, such as the elimination, mitigation, alleviation, or amelioration of symptoms. The pharmaceutically effective amount may be variously determined by factors such as a formulation method, a mode of administration, an age, a body weight, a sex, a pathological condition (severity of the condition) of a patient, a diet, an administration time, an administration interval, a route of administration, an excretion rate, a response sensitivity, a prior therapy, a clinical history, and the like. The dose may be adjusted at the discretion of an attending physician. The pharmaceutically effective amount may be administered at once, or divided and administered in multiple doses of two or more times.

For example, the liquid formulation may be administered once every 2 to 4 weeks over a period of 2 weeks or more at a dose such that the anti-IL-4Rα antibody (e.g., dupilumab) is 300 mg, 250 mg, 200 mg, 150 mg, or 100 mg.

The liquid formulation may be prepared as a general bulk formulation, and components of the liquid formulation are adjusted to a higher concentration than a concentration required for administration, and may be appropriately diluted and used prior to administration.

### Advantageous Effects of Invention

The saccharide-free liquid formulation of an anti-IL-4Rα antibody according to one aspect may have improved stability and improved viscosity compared to an existing commercialized formulation. Therefore, because the liquid formulation has high stability and low viscosity even when it includes the antibody at a high concentration, patient convenience may be increased upon injection. Accordingly, the liquid formulation can be usefully utilized as a medicament for treating an IL-4Rα-related condition.

### Brief Description of Drawings

FIG. 1 is a graph showing the results of measuring viscosity for a saccharide-free formulation and a saccharide-containing formulation.
FIG. 2 is a graph showing the results of measuring the high molecular weight species content (%HMW) for the saccharide-free formulations and the saccharide-containing formulations.
FIG. 3 is a graph showing the results of measuring viscosity for the saccharide-free formulations and the Dupixent formulation.
FIG. 4 is a graph showing the results of measuring the high molecular weight species content (%HMW) for the saccharide-free formulations and the Dupixent formulation.

### Best Mode for Carrying out the Invention

### Mode for the Invention

Hereinafter, the present disclosure will be described in more detail through the following examples. However, these examples are provided for illustrative purposes, and the scope of the present disclosure is not limited to these examples.

### [Experimental Methods]

### 1. Measurement of Viscosity

The viscosity of the sample was measured using a viscometer (manufacturer: RheoSense; model: VROC initium one plus). The analysis was conducted at a temperature of 25 °C during the viscosity measurement. Each sample was measured across 11 segments, and the viscosity of each sample was calculated by averaging the values having a slope fit R² of 0.9995 or greater.

### 2. Analysis of high molecular weight species content (%high molecular weight species, %HMW)

The percentage of high molecular weight species (%HMW) was determined using size exclusion chromatography (SEC) on a Waters HPLC system. The proteins were resolved into three distinct peaks based on molecular weight, corresponding to the HMW peak (referring to protein aggregation), the monomer peak, and the LMW peak (referring to protein degradation) in ascending order of retention time (i.e., in descending order of protein molecular weight). $- % HMW = \left\{area of HMW/area of \left(HMW+monomer+LMW\right)\right\} \times 100$

Each formulation sample for stability testing was stored in a chamber maintaining accelerated stability conditions at a temperature of 25±2 °C and a relative humidity (RH) of 60±5 %.

### [Examples]

### Example 1. Evaluation of Viscosity and %HMW Depending on the Presence or Absence of Saccharide

For a saccharide-containing formulation, after fixing the buffer concentration, the amino acid concentration, and the pH, whether the viscosity and %HMW are improved when the saccharide was removed was evaluated.

Aqueous liquid formulations having the compositions shown in Table 1 below were prepared using dupilumab (CAS No. 1190264-60-8) as an anti-IL-4Rα antibody. For each formulation, the viscosity and the %HMW after storage at 25 °C for 4 weeks were measured.

**[Table 1]**

| No. | Excipient 1 (Saccharide) | Excipient 2 (Amino Acid) | Excipient 3 (Amino Acid) | Viscosity (cP) | %HMW (25 °C, 4 weeks) |
|---|---|---|---|---|---|
| 1 | **Saccharide-free** | **120 mM arginine** | - | **8.1** | **2.93** |
| 2 | 4.3% (w/v) trehalose | 70 mM arginine | - | 12.7 | 3.14 |
| 3 | 1.8% (w/v) sorbitol | 70 mM arginine | - | 11.6 | 3.21 |
| 4 | 1.8% (w/v) mannitol | 70 mM arginine | - | 14.2 | 3.38 |
| 5 | **Saccharide-free** | **120 mM arginine** | **30 mM lysine** | **7.9** | **2.80** |
| 6 | 4.3% (w/v) trehalose | 70 mM arginine | 30 mM lysine | 10.8 | 2.93 |
| 7 | 1.8% (w/v) sorbitol | 70 mM arginine | 30 mM lysine | 11.9 | 3.02 |
| 8 | 1.8% (w/v) mannitol | 70 mM arginine | 30 mM lysine | 13.5 | 3.10 |
| 9 | **Saccharide-free** | **120 mM arginine** | **30 mM methionine** | **7.8** | **2.59** |
| 10 | 4.3% (w/v) trehalose | 70 mM arginine | 30 mM methionine | 11.5 | 2.71 |
| 11 | 1.8% (w/v) sorbitol | 70 mM arginine | 30 mM methionine | 11.2 | 2.76 |
| 12 | 1.8% (w/v) mannitol | 70 mM arginine | 30 mM methionine | 14.9 | 2.94 |
| 13 | **Saccharide-free** | **145 mM histidine** | - | **8.7** | **2.26** |
| 14 | 4.3% (w/v) trehalose | 80 mM histidine | - | 16.8 | 2.59 |
| 15 | 1.8% (w/v) sorbitol | 80 mM histidine | - | 14.8 | 2.58 |
| 16 | 1.8% (w/v) mannitol | 80 mM histidine | - | 13.8 | 2.56 |
| 17 | **Saccharide-free** | **145 mM histidine** | **30 mM lysine** | **8.8** | **2.19** |
| 18 | 4.3% (w/v) trehalose | 80 mM histidine | 30 mM lysine | 11.1 | 2.49 |
| 19 | 1.8% (w/v) sorbitol | 80 mM histidine | 30 mM lysine | 12.8 | 2.51 |
| 20 | 1.8% (w/v) mannitol | 80 mM histidine | 30 mM lysine | 15.3 | 2.49 |
| 21 | **Saccharide-free** | **145 mM histidine** | **30 mM methionine** | **9.4** | **2.09** |
| 22 | 4.3% (w/v) trehalose | 80 mM histidine | 30 mM methionine | 12.4 | 2.33 |
| 23 | 1.8% (w/v) sorbitol | 80 mM histidine | 30 mM methionine | 13.0 | 2.34 |
| 24 | 1.8% (w/v) mannitol | 80 mM histidine | 30 mM methionine | 13.4 | 2.35 |
| 25 | **Saccharide-free** | **220 mM glycine** | - | **14.9** | **3.62** |
| 26 | 4.3% (w/v) trehalose | 120 mM glycine | - | 23.5 | 3.69 |
| 27 | 1.8% (w/v) sorbitol | 120 mM glycine | - | 43.2 | 3.72 |
| 28 | 1.8% (w/v) mannitol | 120 mM glycine | - | 35.8 | 3.69 |
| 29 | **Saccharide-free** | **220 mM glycine** | **30 mM lysine** | **10.8** | **3.41** |
| 30 | 4.3% (w/v) trehalose | 120 mM glycine | 30 mM lysine | 31.4 | 3.68 |
| 31 | 1.8% (w/v) sorbitol | 120 mM glycine | 30 mM lysine | 38.2 | 3.79 |
| 32 | 1.8% (w/v) mannitol | 120 mM glycine | 30 mM lysine | 22.0 | 3.68 |
| 33 | **Saccharide-free** | **220 mM glycine** | **30 mM methionine** | **16.8** | **3.10** |
| 34 | 4.3% (w/v) trehalose | 120 mM glycine | 30 mM methionine | 27.6 | 3.25 |
| 35 | 1.8% (w/v) sorbitol | 120 mM glycine | 30 mM methionine | 38.0 | 3.26 |
| 36 | 1.8% (w/v) mannitol | 120 mM glycine | 30 mM methionine | 30.9 | 3.23 |

| | | | | | |
|---|---|---|---|---|---|
| ^{a} All formulations contain 175 mg/mL dupilumab, 10 mM sodium acetate, and 0.2% (w/v) polysorbate 80, and have a pH of 6.0. | | | | | |

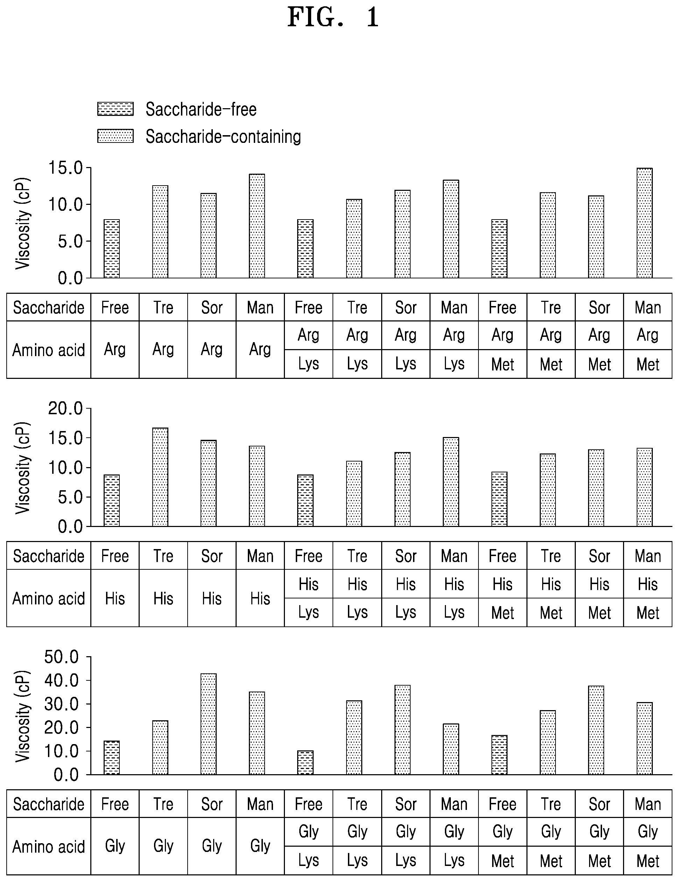

FIG. 1 is a graph showing the results of measuring viscosity for the saccharide-free formulations and the saccharide-containing formulations.

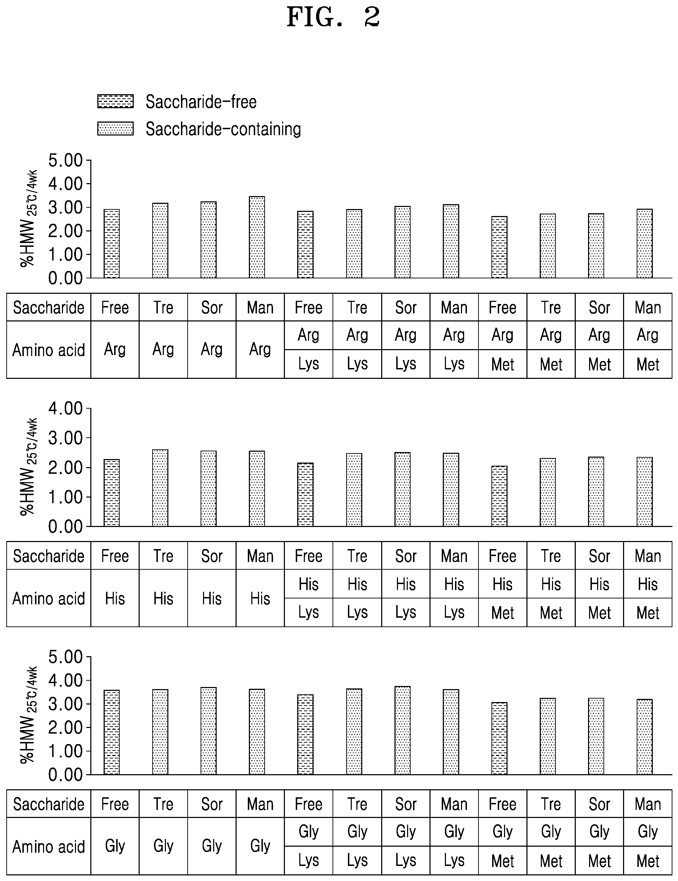

FIG. 2 is a graph showing the results of measuring the high molecular weight species content (%HMW) for the saccharide-free formulations and the saccharide-containing formulations.

As a result, as shown in Table 1, FIG. 1, and FIG. 2, upon removing the saccharide from the saccharide-containing formulations while keeping the buffer concentration, amino acid concentration, surfactant concentration, and pH constant, it was confirmed that the viscosity and %HMW_{25 °C/4 weeks} were simultaneously improved.

### Example 2. Comparative Evaluation of Viscosity and %HMW of Saccharide-Free Formulations and Dupixent

As shown in Table 2 below, the viscosity and the %HMW immediately after preparation (Day 0, Initial) were measured for 25 types of saccharide-free formulations having different buffer concentrations, amino acid concentrations, and pH values and for the saccharide-containing Dupixent formulation.

**[Table 2]**

| No.^{a} | Buffer | pH | Excipient 1 (Saccharide) | Excipient 2 (Amino Acid) | Excipient 3 (Amino Acid) | Viscosity %HMW (Initial) | %HMW (Initial) |
|---|---|---|---|---|---|---|---|
| 37 | 6 mM sodium acetate | 5.9 | Saccharide-free | 145 mM histidine | 30 mM methionine | 8.1 | 1.76 |
| 38 | 8 mM sodium acetate | 5.6 | | 115 mM histidine | 25 mM methionine | 7.6 | 1.71 |
| 39 | | 6.2 | | | 25 mM methionine | 9.7 | 1.92 |
| 40 | | 5.6 | | | 35 mM methionine | 8.0 | 1.73 |
| 41 | | 6.2 | | | 35 mM methionine | 9.9 | 1.88 |
| 42 | | 5.6 | | 175 mM histidine | 25 mM methionine | 7.7 | 1.69 |
| 43 | | 6.2 | | | 25 mM methionine | 8.0 | 1.78 |
| 44 | | 5.6 | | | 35 mM methionine | 7.7 | 1.62 |
| 45 | | 6.2 | | | 35 mM methionine | 8.5 | 1.81 |
| 46 | 10 mM sodium acetate | 5.9 | | 85 mM histidine | 30 mM methionine | 9.5 | 1.84 |
| 47 | | 5.3 | | 145 mM histidine | 30 mM methionine | 7.4 | 1.64 |
| 48 | | 5.9 | | | 30 mM methionine | 8.0 | 1.75 |
| 49 | | 6.5 | | | 30 mM methionine | 10.0 | 1.98 |
| 50 | | 5.9 | | | 20 mM methionine | 8.0 | 1.7 |
| 51 | | 5.9 | | | 40 mM methionine | 8.3 | 1.78 |
| 52 | | 5.9 | | 205 mM histidine | 30 mM methionine | 7.4 | 1.62 |
| 53 | 12 mM sodium acetate | 5.6 | | 115 mM histidine | 25 mM methionine | 8.2 | 1.71 |
| 54 | | 6.2 | | | 25 mM methionine | 8.9 | 1.90 |
| 55 | | 5.6 | | | 35 mM methionine | 7.9 | 1.72 |
| 56 | | 6.2 | | | 35 mM methionine | 9.1 | 1.92 |
| 57 | | 5.6 | | 175 mM histidine | 25 mM methionine | 8.0 | 1.66 |
| 58 | | 6.2 | | | 25 mM methionine | 8.1 | 1.83 |
| 59 | | 5.6 | | | 35 mM methionine | 8.1 | 1.69 |
| 60 | | 6.2 | | | 35 mM methionine | 8.6 | 1.81 |
| 61 | 14 mM sodium acetate | 5.9 | | 145 mM histidine | 30 mM methionine | 8.3 | 1.74 |
| 62^{b} | 12.5 mM sodium acetate + 20 mM histidine | 5.9 | 5% (w/v) sucrose | 50 mM arginine | - | 11.1 | 2.03 |

| | | | | | | | |
|---|---|---|---|---|---|---|---|
| ^{a} All formulations contain 175 mg/mL dupilumab and 0.2% (w/v) polysorbate 80. ^{b} Formulation 62 is the Dupixent formulation. | | | | | | | |

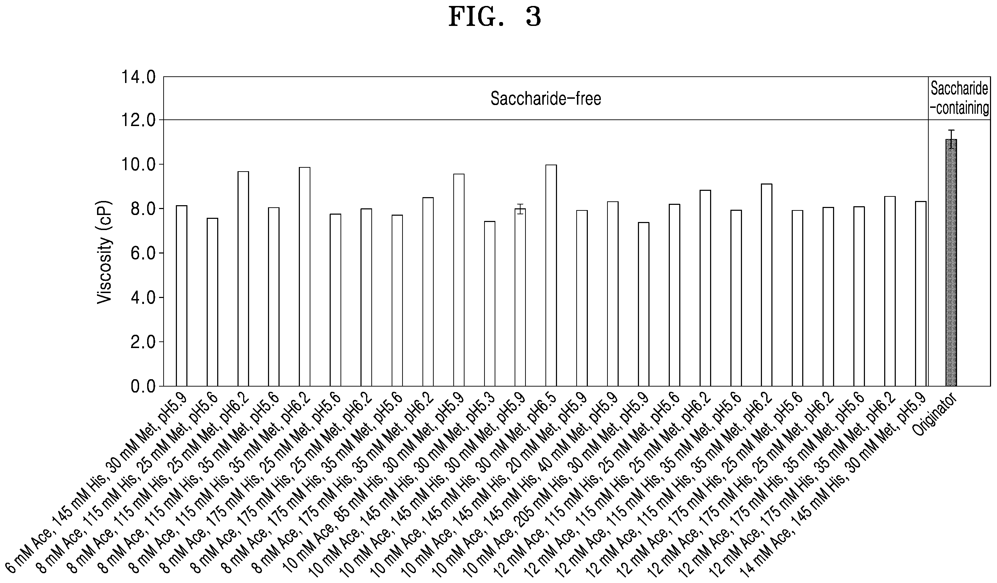

FIG. 3 is a graph showing the results of measuring viscosity for the saccharide-free formulations and the Dupixent formulation.

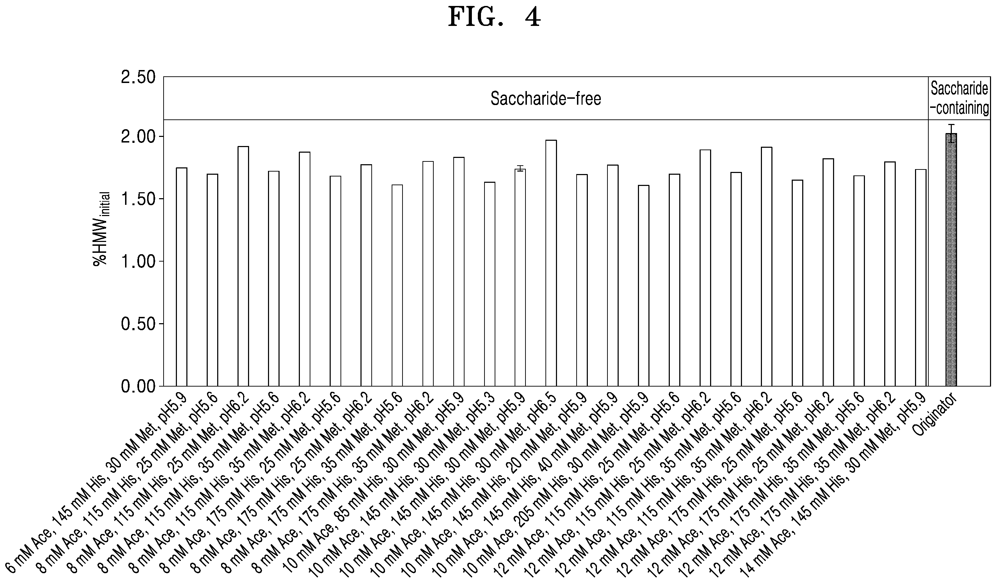

FIG. 4 is a graph showing the results of measuring the high molecular weight species content (%HMW) for the saccharide-free formulations and the Dupixent formulation.

As a result, as shown in Table 2, FIG. 3, and FIG. 4, in the saccharide-free formulations including 6 to 14 mM sodium acetate, 85 to 205 mM histidine, and 20 to 40 mM methionine and having a pH of 5.3 to 6.5, it was confirmed that the viscosity and %HMW were simultaneously improved compared to the Dupixent formulation (Formulation 62).

The above description of the present disclosure is provided for the purpose of illustration, and those of ordinary skill in the art to which the present disclosure pertains will understand that the present disclosure may be easily modified into other specific forms without changing the technical spirit or essential features thereof. Therefore, it should be understood that the embodiments described above are illustrative in all respects and not restrictive.

## Claims

1. A liquid formulation comprising:
an anti-IL-4Rα antibody;
a buffer; and
an amino acid,
wherein the liquid formulation is free of saccharide and
has a pH of about 4.5 to about 6.5.

2. The liquid formulation of claim 1, wherein the anti-IL-4Rα antibody is dupilumab.

3. The liquid formulation of claim 1, wherein a concentration of the anti-IL-4Rα antibody is about 5 mg/mL to about 300 mg/mL.

4. The liquid formulation of claim 1, wherein a concentration of the anti-IL-4Rα antibody is about 100 mg/mL to about 200 mg/mL.

5. The liquid formulation of claim 1, wherein a concentration of the anti-IL-4Rα antibody is about 125 mg/mL to about 200 mg/mL.

6. The liquid formulation of claim 1, wherein the buffer comprises one or more selected from acetate, phosphate, citrate, succinate, malate, tartrate, and carbonate.

7. The liquid formulation of claim 1, wherein the buffer comprises acetate.

8. The liquid formulation of claim 1, wherein the buffer is sodium acetate.

9. The liquid formulation of any one of claims 1 to 8, wherein a concentration of the buffer is about 5 mM to about 50 mM.

10. The liquid formulation of claim 7, wherein a concentration of the acetate is about 6 mM to about 14 mM.

11. The liquid formulation of claim 7, wherein a concentration of the acetate is about 20 mM to about 40 mM.

12. The liquid formulation of claim 1, wherein the amino acid comprises one or more selected from arginine, histidine, glycine, methionine, and lysine.

13. The liquid formulation of claim 1, wherein the amino acid is selected from the following:
1) arginine;
2) a combination of arginine and lysine;
3) a combination of arginine and methionine;
4) histidine;
5) a combination of histidine and lysine;
6) a combination of histidine and methionine;
7) glycine;
8) a combination of glycine and lysine;
9) a combination of glycine and methionine; or
10) a combination of histidine, arginine, and methionine.

14. The liquid formulation of claim 12 or 13, wherein a concentration of the arginine is about 75 mM to about 165 mM.

15. The liquid formulation of claim 12 or 13, wherein a concentration of the histidine is about 85 mM to about 205 mM.

16. The liquid formulation of claim 12 or 13, wherein a concentration of the histidine is about 30 mM to about 70 mM.

17. The liquid formulation of claim 12 or 13, wherein a concentration of the glycine is about 125 mM to about 315 mM.

18. The liquid formulation of claim 12 or 13, wherein a concentration of the methionine is about 10 mM to about 40 mM.

19. The liquid formulation of claim 12 or 13, wherein a concentration of the lysine is about 20 mM to about 40 mM.

20. The liquid formulation of claim 1, wherein the amino acid is a combination of histidine and methionine.

21. The liquid formulation of claim 20, wherein a concentration of the histidine is about 85 mM to about 205 mM, and a concentration of the methionine is about 20 mM to about 40 mM.

22. The liquid formulation of claim 1, wherein the amino acid is a combination of histidine, arginine, and methionine.

23. The liquid formulation of claim 22,
wherein a concentration of the histidine is about 30 mM to about 70 mM, a concentration of the arginine is about 80 mM to about 120 mM, and
a concentration of the methionine is about 10 mM to about 40 mM.

24. The liquid formulation of any one of claims 1 to 23, further comprising a surfactant.

25. The liquid formulation of claim 24, wherein the surfactant comprises a nonionic surfactant.

26. The liquid formulation of claim 24, wherein the surfactant comprises polysorbate 20, polysorbate 80, or a combination thereof.

27. The liquid formulation of any one of claims 24 to 26, wherein a concentration of the surfactant is 0.01% (w/v) to 0.9% (w/v).

28. The liquid formulation of any one of claims 24 to 26, wherein a concentration of the surfactant is about 0.1% (w/v) to about 0.3% (w/v).

29. The liquid formulation of any one of claims 1 to 28, wherein a viscosity of the liquid formulation is decreased by 5 % or more compared to a viscosity of a saccharide-containing formulation.

30. The liquid formulation of any one of claims 1 to 28, wherein a viscosity of the liquid formulation is less than 17.0 cP.

31. The liquid formulation of any one of claims 1 to 28, wherein a viscosity of the liquid formulation is less than 11.1 cP.

32. The liquid formulation of any one of claims 1 to 28, wherein a high molecular weight species content (%HMW) of the antibody in the liquid formulation measured after storage at 25 °C for 4 weeks is decreased by 1 % or more compared to a saccharide-containing formulation.

33. The liquid formulation of any one of claims 1 to 28, wherein a high molecular weight species content (%HMW) of the antibody measured after storage at 25 °C for 4 weeks is less than 3.70 %.

34. The liquid formulation of any one of claims 1 to 28, wherein a high molecular weight species content (%HMW) of the antibody in the liquid formulation measured immediately after preparation is decreased by 2 % or more compared to a saccharide-containing formulation.

35. The liquid formulation of any one of claims 1 to 28, wherein a high molecular weight species content (%HMW) of the antibody measured immediately after preparation is less than 2.03 %.

36. A liquid formulation comprising:
about 100 mg/mL to about 200 mg/mL of dupilumab;
about 6 mM to about 14 mM of acetate;
about 20 mM to about 355 mM of an amino acid, the amino acid comprising one or more selected from arginine, histidine, glycine, methionine, and lysine; and
about 0.1% (w/v) to about 0.9% (w/v) of polysorbate 80,
wherein the liquid formulation is free of saccharide and has a pH of about 5.3 to about 6.5.

37. A liquid formulation comprising:
about 100 mg/mL to about 200 mg/mL of dupilumab;
about 6 mM to about 14 mM of acetate;
about 85 mM to about 205 mM of histidine;
about 20 mM to about 40 mM of methionine; and
about 0.1% (w/v) to about 0.9% (w/v) of polysorbate 80,
wherein the liquid formulation is free of saccharide and has a pH of about 5.3 to about 6.5.

38. A liquid formulation comprising:
about 100 mg/mL to about 200 mg/mL of dupilumab;
about 20 mM to about 40 mM of acetate;
about 40 mM to about 60 mM of histidine;
about 80 mM to about 120 mM of arginine;
about 10 mM to about 40 mM of methionine; and
about 0.1% (w/v) to about 0.9% (w/v) of polysorbate 80,
wherein the liquid formulation is free of saccharide and has a pH of about 4.5 to about 5.9.

39. The liquid formulation of any one of claims 1 to 38, wherein the liquid formulation is for subcutaneous injection.

40. A device comprising the liquid formulation of any one of claims 1 to 39.

41. The device of claim 40, wherein the liquid formulation is contained in a container selected from a syringe, a pre-filled syringe, a pre-filled pen, a microinfuser, an autoinjector, a bottle, a vial, and a tube.

42. A method of treating an IL-4Rα-related condition, comprising administering the liquid formulation of any one of claims 1 to 39 to a subject in need thereof.

43. The method of treating an IL-4Rα-related condition of claim 42, wherein the IL-4Rα-related condition is an IL-4-mediated disease or an IL-13-mediated disease.

44. The method of treating an IL-4Rα-related condition of claim 42, wherein the IL-4Rα-related condition is any one selected from atopic dermatitis, asthma, chronic rhinosinusitis (CRS), chronic rhinosinusitis with nasal polyposis (CRSwNP), allergic fungal rhinosinusitis (AFRS), eosinophilic esophagitis (EoE), prurigo nodularis (PN), bullous pemphigoid (BP), chronic spontaneous urticaria (CSU), chronic inducible urticaria (CIndU), and chronic obstructive pulmonary disease (COPD).

45. Use of a liquid formulation according to any one of claims 1 to 39 in the manufacture of a medicament for treating an IL-4Rα-related condition
